# EUROPÄISCHE PATENTANMELDUNG

(11) **EP 3 821 942 A1**
(43) Veröffentlichungstag der Anmeldung: **19.05.2021**
(21) Anmeldenummer: 19209653.5
(22) Anmeldetag: 18.11.2019
(51) Int. Cl.: A61N 1/37, A61N 1/368

(54) **IMPLANTIERBARE ANORDNUNG ZUR STIMULATION EINES MENSCHLICHEN ODER TIERISCHEN HERZENS**

(71) Anmelder: BIOTRONIK SE & Co. KG, 12359 Berlin (DE)
(72) Erfinder: Dörr, Thomas, 12437 Berlin (DE)
(74) Vertreter: Yang, Tian

(57) **Zusammenfassung**

Die Erfindung betrifft eine implantierbare Anordnung zur Stimulation eines menschlichen oder tierischen Herzens, aufweisend einen Prozessor, eine Speichereinheit, eine Stimulationseinheit zum Stimulieren eines His-Bündels oder eines anderen kardialen Bereichs eines menschlichen oder tierischen Herzens, eine Detektionseinheit zum Detektieren eines elektrischen Signals desselben Herzens und eine Diagnoseeinheit zur Überprüfung von Funktionsparametern der implantierbaren Anordnung. Diese Anordnung führt in ihrem Betrieb folgendes Programm aus: a) Erkennen mittels der Diagnoseeinheit, ob mindestens ein Störzustand der implantierbaren Anordnung vorliegt; b) Überprüfen (220), ob eine zur Stimulation eines His-Bündels eines menschlichen oder tierischen Herzens vorgesehene Elektrode an die Stimulationseinheit angeschlossen ist; c) Umschalten eines Betriebszustands der implantierbaren Anordnung in einen Sicherheitsmodus, wenn ein Störzustand erkannt wurde, wobei der Sicherheitsmodus zumindest aus einem ersten Sicherheitsmodus (230) und einem zweiten Sicherheitsmodus (240) ausgewählt wird, wobei i) der erste Sicherheitsmodus (230) ausgewählt wird, wenn das in Schritt b) erfolgte Überprüfen (220) ergeben hat, dass keine zur Stimulation eines His-Bündels eines menschlichen oder tierischen Herzens vorgesehene Elektrode an die Stimulationseinheit angeschlossen ist, und ii) der zweite Sicherheitsmodus (240) ausgewählt wird, wenn das in Schritt b) erfolgte Überprüfen (220) ergeben hat, dass eine zur Stimulation eines His-Bündels eines menschlichen oder tierischen Herzens vorgesehene Elektrode an die Stimulationseinheit angeschlossen ist.

## Beschreibung

Die vorliegende Erfindung betrifft eine implantierbare Anordnung zur Stimulation eines menschlichen oder tierischen Herzens gemäß dem Oberbegriff des Anspruchs 1, ein Verfahren zur Steuerung des Betriebs einer derartigen implantierbaren Anordnung gemäß dem Oberbegriff des Anspruchs 11 und ein Computerprogrammprodukt, das zur Durchführung eines derartigen Verfahrens geeignet ist, gemäß dem Oberbegriff des Anspruchs 12.

Implantierbare Anordnungen zur Stimulation eines menschlichen oder tierischen Herzens, wie etwa Herzschrittmacher, sind seit langem bekannt. Sie können unterschiedliche Funktionen ausführen. Dabei können verschiedene Stimulationsprogramme von einem entsprechenden Herzschrittmacher durchgeführt werden, um das behandelte Herz wieder in einen normalen Zustand zu bringen. Es sind auch Herzschrittmacher zur Stimulation des His-Bündels bekannt.

Das His-Bündel ist ein Bündel spezifischer Herzmuskelzellen, das Teil des kardialen Erregungsleitungssystems ist. Das His-Bündel liegt distal des atrioventrikulären Knotens in Richtung auf die Herzspitze.

Häufig werden zur His-Bündel-Stimulation konventionelle Herzschrittmacher und implantierbare Defibrillatoren eingesetzt. Dabei wird ein Stimulationsausgang eines derartigen konventionellen Geräts mit einer His-Bündel-Elektrode verbunden. Die konventionellen Geräte werden dann typischerweise im Rahmen der verfügbaren Parameterwertebereiche unter Eingehen von Kompromissen an die Bedürfnisse einer His-Bündel-Stimulation angepasst.

Konventionelle Geräte zur Stimulation des menschlichen oder tierischen Herzens weisen regelmäßig einen Sicherheitsmodus (auch als Backup-Modus bezeichnet) auf. Wenn seitens des Gerätes ein Fehler detektiert wird, wird der Sicherheitsmodus aktiviert, in dem typischerweise festgelegte Geräteeinstellungen für den weiteren Gerätebetrieb verwendet werden. Mit den klassischerweise genutzten Geräteeinstellungen ist in der Regel jedoch keine hinreichende His-Bündel-Stimulation möglich. Beispielsweise ist es möglich, dass eine His-Bündel-Elektrode an den rechtsatrialen Anschluss eines Geräts zur Stimulation des Herzens angeschlossen ist, für dieses Gerät aber ein VVI-Modus mit rechtsventrikulärer Stimulation Sicherheitsmodus hinterlegt ist. Im VVI-Modus werden lediglich rechtsventrikuläre Signale detektiert. Darüber hinaus erfolgt lediglich eine rechtsventrikuläre Stimulation. Ferner wird im VVI-Modus bei einer detektierten herzeigenen Aktivität eine Stimulation durch den Herzschrittmacher inhibiert. Der rechtsatriale Anschluss wird im VVI-Modus jedoch gar nicht mehr angesprochen. Mithin kann keine His-Bündel-Stimulation mehr erfolgen, sodass der Patient, der ein derartiges Gerät trägt, dann unterversorgt ist.

Die US 6,996,437 B2 beschreibt verschiedene Sicherheitsmodi eines Herzschrittmachers, der für eine ventrikuläre Stimulation eines Herzens ausgelegt ist.

Die US 8,688,234 B2 beschreibt einen Herzschrittmacher, der dazu geeignet ist, das His-Bündel eines Patienten zu stimulieren. Dabei sind verschiedene Stimulationsmodi vorgesehen.

Der vorliegenden Erfindung liegt die Aufgabe zugrunde, ein Gerät zur Stimulation eines menschlichen oder tierischen Herzens anzugeben, das auch im Fehlerfall in der Lage ist, eine verlässliche His-Bündel-Stimulation durchführen zu können.

Diese Aufgabe wird durch eine implantierbare Anordnung zur Stimulation eines menschlichen oder tierischen Herzens mit den Merkmalen des Anspruchs 1 gelöst.

Eine derartige Anordnung weist einen Prozessor, eine Speichereinheit (Speichereinrichtung) und eine Stimulationseinheit (Stimulationseinrichtung) zum Stimulieren eines His-Bündels oder eines anderen kardialen Bereichs eines menschlichen oder tierischen Herzens auf. Darüber hinaus weist eine derartige Anordnung eine Detektionseinheit (Detektionseinrichtung) zum Detektieren eines elektrischen Signals desselben Herzens auf. Ferner ist eine Diagnoseeinheit (Diagnoseeinrichtung) zur Überprüfung von Funktionsparametern der implantierbaren Anordnung vorgesehen.

Die implantierbare Anordnung zeichnet sich erfindungsgemäß dadurch aus, dass die Speichereinheit ein computerlesbares Programm aufweist, das den Prozessor dazu veranlasst, die nachfolgend erläuterten Schritte auszuführen, wenn es auf dem Prozessor ausgeführt wird.

So wird einerseits mittels der Diagnoseeinheit erkannt, ob mindestens ein Störzustand der implantierbaren Anordnung vorliegt.

Andererseits wird überprüft, ob eine zur Stimulation eines His-Bündels eines menschlichen oder tierischen Herzens vorgesehene Elektrode an die Stimulationseinheit angeschlossen ist. Mit anderen Worten wird überprüft, ob die implantierbare Anordnung zur Stimulation des His-Bündels eines Patienten vorgesehen ist.

Wenn ein Störzustand erkannt wurde, wird der Betriebszustand der implantierbaren Anordnung gemäß dem ausgeführten Programm in einen Sicherheitsmodus (auch als Backup-Modus bezeichnet) umgeschaltet. Dieser Sicherheitsmodus kann zumindest aus einem ersten und einem zweiten Sicherheitsmodus ausgewählt werden.

Der erste Sicherheitsmodus wird dann ausgewählt, wenn bei der Überprüfung, ob eine zur Stimulation des His-Bündels des Herzens vorgesehene Elektrode angeschlossen ist, ermittelt wurde, dass keine derartige Elektrode angeschlossen ist. Dabei sorgt der erste Sicherheitsmodus für einen ersten Sicherheitsbetrieb der implantierbaren Anordnung, in dem die implantierbare Anordnung keine spezifischen Anforderungen einer His-Bündel-Stimulation erfüllt. Denn wenn keine zur Stimulation des His-Bündels vorgesehene Elektrode an die Stimulationseinheit der implantierbaren Anordnung angeschlossen ist, wird auch kein auf eine His-Bündel-Stimulation speziell zugeschnittener Sicherheitsbetrieb benötigt. Vielmehr kann als erster Sicherheitsmodus beispielsweise ein aus dem Stand der Technik bekannter Sicherheitsmodus verwendet werden, wie beispielsweise ein VVI-Modus.

Wenn bei der Überprüfung, ob eine zur Stimulation des His-Bündels geeignete Elektrode an die Stimulationseinheit angeschlossen ist, hingegen ermittelt wurde, dass dies der Fall ist, wird der zweite Sicherheitsmodus ausgewählt. Der zweite Sicherheitsmodus sorgt dabei für einen zweiten Sicherheitsbetrieb der implantierbaren Anordnung. In diesem zweiten Sicherheitsbetrieb ist die implantierbare Anordnung in der Lage, spezifische Anforderungen einer His-Bündel-Stimulation zu erfüllen. Beispielsweise kann in dem durch den zweiten Sicherheitsmodus bedingten zweiten Sicherheitsbetrieb ein Stimulationspuls, der eine Amplitude aufweist, die in einem Bereich von 7,5 V bis 30 V, insbesondere von 12 V bis 30 V, insbesondere von 15 V bis 25 V, insbesondere von 20 V bis 22 V liegt, und der eine Pulsbreite aufweist, die in einem Bereich von 1 ms bis 15 ms, insbesondere von 2 ms bis 12 ms, insbesondere von 3 ms bis 10 ms, insbesondere von 4 ms bis 9 ms, insbesondere von 5 ms bis 8 ms liegt, weiterhin von demjenigen Anschluss der Stimulationseinheit durchgeführt werden, an dem die zur Stimulation des His-Bündels vorgesehene Elektrode angeschlossen ist.

Mithin ist die vorliegend beanspruchte implantierbare Anordnung derart ausgelegt, dass sie unterschiedliche Sicherheitsmodi in Abhängigkeit davon, ob eine zur His-Bündel-Stimulation geeignete Elektrode an die Stimulationseinheit der Anordnung angeschlossen ist oder nicht, auswählen und anwenden kann. Dadurch kann einerseits für den Fall, dass keine zur His-Bündel-Stimulation geeignete Elektrode angeschlossen ist, wie bislang ein Sicherheitsmodus (nämlich der erste Sicherheitsmodus)ausgewählt werden, der einem herkömmlichen Sicherheitsmodus entspricht und eine einfache Grundfunktion der implantierbaren Anordnung gewährleistet. Auf der anderen Seite kann aber dann, wenn eine zur His-Bündel-Stimulation geeignete Elektrode an die Stimulationseinheit der implantierbaren Anordnung angeschlossen ist, ein anderer Sicherheitsmodus (nämlich der zweite Sicherheitsmodus) ausgewählt werden, der zwar ebenfalls die Grundfunktionen des herkömmlichen Sicherheitsmodus erfüllt, gleichzeitig aber weiterhin eine His-Bündel-Stimulation zulässt. Auf diese Weise wird eine bei den aus dem Stand der Technik bekannten Herzschrittmachern auftretende Unterversorgung eines Patienten vermieden. Somit ermöglicht der zweite Sicherheitsmodus die Abgabe einer lebenserhaltenden Stimulationstherapie eines His-Bündel-Stimulator auch dann, wenn durch die Detektion einer Störfunktion ein allgemeiner oder sonstiger Betriebszustand in einen Sicherheitsmodus bzw. Sicherheitsbetrieb der implantierbaren Anordnung umgeschaltet wird.

Die implantierbare Anordnung ist in einer Variante als Herzschrittmacher ausgestaltet. In einer weiteren Variante ist sie als implantierbarer Kardioverter/Defibrillator (ICD) ausgestaltet. In einer weiteren Variante ist die Anordnung als Gerät zur Durchführung einer kardialen Resynchronisation (CRT) ausgestaltet. In einer weiteren Variante ist die implantierbare Anordnung als Gerät zur kardialen Resynchronisation mit ICD-Funktion ausgestaltet (CRT-D-Gerät). In einer weiteren Variante ist die Anordnung als implantierbarer sondenloser Herzschrittmacher (sogenannter implantable leadless pacer, iLP) ausgestaltet.

In einer Variante ist die Information, die für das Umschalten des Betriebszustandes der Anordnung in den Sicherheitsmodus benötigt wird, in einem nicht flüchtigen Speicher, einem redundanten Speicher oder einem wiederherstellbaren Speicher der implantierbaren Anordnung hinterlegt. Dabei kann es sich bei diesem Speicher um die Speichereinheit der implantierbaren Anordnung, um einen Speicherbereich der Speichereinheit der implantierbaren Anordnung oder um einen von der Speichereinheit separaten Speicher handeln. Die Hinterlegung der für das Umschalten benötigten Informationen in einem nicht flüchtigen Speicher ist dabei eine besonders geeignete Variante. Besonders geeignete nicht flüchtige Speicher sind Speicher des Typs ROM, EPROM, EEPROM, FLASH. Dann kann besonders einfach sichergestellt werden, dass die für das Umschalten in den Sicherheitsmodus benötigten Informationen nicht versehentlich gelöscht werden können, sondern stets abrufbereit sind. Dies erhöht die Gerätesicherheit.

In einer Variante ist die implantierbare Anordnung dazu in der Lage, automatisch zu erkennen, ob eine zur His-Bündel-Stimulation vorgesehene Elektrode an die Stimulationseinheit der Anordnung angeschlossen ist. Dies kann beispielsweise über das Auslesen einer Kodierung eines Anschlusssteckers der zur His-Bündel-Stimulation vorgesehenen Elektrode realisiert werden.

In einer weiteren Variante erfolgt das Überprüfen, ob eine zur Stimulation des His-Bündels eines menschlichen oder tierischen Herzens vorgesehene Elektrode an die Stimulationseinheit angeschlossen ist, mittels einer Information, die in der Speichereinheit oder einem anderen Speicher der implantierbaren Anordnung abgespeichert ist. Beispielsweise kann eine derartige Information im Rahmen der Implantation der implantierbaren Anordnung in der Speichereinheit hinterlegt werden. Dann ist es nicht erforderlich, dass ein Anschlussstecker einer zur His-Bündel-Stimulation geeigneten Elektrode auf besondere Art und Weise ausgestaltet ist. Vielmehr kann ein Operateur dann, wenn er eine zur His-Bündel-Stimulation vorgesehenen Elektrode zusammen mit der implantierbaren Anordnung implantiert, in der implantierbaren Anordnung hinterlegen, dass diese Anordnung mit einer derartigen His-Bündel-Stimulationselektrode ausgestattet ist. In dieser Variante ist es lediglich erforderlich, im Speicher der implantierbaren Anordnung abzufragen, ob eine entsprechende Information hinterlegt ist. Dann wird davon ausgegangen, dass eine derartige zur His-Bündel-Stimulation geeignete Elektrode auch tatsächlich angeschlossen ist, sodass nachfolgend in einem Störfall ein Umschalten des Betriebszustands der implantierbaren Anordnung in den zweiten Sicherheitsmodus erfolgt.

In einer Variante ist die Diagnoseeinheit derart ausgelegt, dass sie anhand bestimmter Ereignisse erkennen kann, ob ein Störzustand der implantierbaren Anordnung vorliegt oder nicht. Zu diesen Ereignissen, bei denen ein Störzustand erkannt wird, zählen in dieser Variante ein Speicherfehler (wie beispielsweise ein sogenanntes Single Bit Upset), ein Softwarelaufzeitfehler, ein Softwarestatusfehler, ein erkannter Cyberangriff (also der Versuch eines unautorisierten Fernzugriffs auf die implantierbare Anordnung), ein Parameterfehler, ein Aktivieren eines Reset-Zustandes der implantierbaren Anordnung (wobei grundsätzlich unterschiedliche Reset-Zustände der implantierbaren Anordnung denkbar und einstellbar sind), eine Batterieerschöpfung (also ein Erreichen oder ein Unterschreiten einer vorgebbaren Kapazitätsgrenze einer Batterie der implantierbaren Anordnung, bei der ein fehlerfreier Betrieb der Anordnung nicht mehr gewährleistet ist) und eine externe elektromagnetische Störung der implantierbaren Anordnung.

Eine solche externe elektromagnetische Störung kann beispielsweise ein starkes Permanentmagnetfeld sein, wie es von Magnetresonanztomographie-(MRT)-Geräten abgestrahlt wird. Wenn beispielsweise ein Patient weiß, dass innerhalb eines bestimmten Zeitraums eine MRT-Untersuchung bei ihm durchgeführt werden soll, kann ein Arzt eine entsprechende Information in der implantierbaren Anordnung hinterlegen. Da starke Magnetfelder die Funktionsfähigkeit der implantierbaren Anordnung beeinträchtigen können, ist die implantierbare Anordnung dann während eines vorgebbaren Zeitraums mit einer vergrößerten Überwachungsgenauigkeit auf die Detektion sensibilisiert, ob die Anordnung einem Permanentmagnetfeld ausgesetzt ist. Sollte dies der Fall sein, kann unmittelbar einer der Sicherheitsmodi ausgewählt und aktiviert werden, um eine Fehlfunktion der implantierbaren Anordnung zu verhindern. Mithin kann die Detektion einer derartigen MRT-Umgebung als Störzustand erkannt werden und zur Aktivierung eines der Sicherheitsmodi führen. Nach Abschluss der MRT-Untersuchung kann die implantierbare Anordnung dann wieder manuell in ihren gewöhnlichen Betriebszustand überführt werden.

In einer Variante ist der zweite Sicherheitsbetrieb durch mindestens eine der nachfolgend erläuterten Konfigurationen gekennzeichnet.

Beispielsweise kann während des zweiten Sicherheitsbetriebs als Konfiguration eine spezifische His-Bündel-Stimulations-Einstellung eines Elektrodenanschlusses der Stimulationseinheit vorgenommen werden, an den eine zur His-Bündel-Stimulation vorgesehene Elektrode angeschlossen ist. Die Auswahl des Elektrodenanschlusses, an den die His-Bündel-Elektrode angeschlossen ist, kann dabei - wie oben beschrieben - beispielsweise durch eine benutzerabhängige Information erfolgen.

Alternativ oder zusätzlich kann eine Stimulationsenergie einer Stimulation, die von einer His-Bündel Elektrode zur Stimulation eines His-Bündels abgegeben werden kann, spezifisch derart eingestellt werden, dass sie sich zur Stimulation des His-Bündels des betreffenden Herzens eignet. Geeignete Stimulationsenergien (gekennzeichnet durch Spannung und Pulsbreiten) sind weiter oben angegeben.

Alternativ oder zusätzlich kann eine Detektionsempfindlichkeit (Wahrnehmungsempfindlichkeit) der Detektionseinheit für eine Detektion His-Bündel-spezifischer Signale eingestellt werden. Dann kann sichergestellt werden, dass His-Bündelspezifische Signale auch tatsächlich im Sicherheitsbetrieb korrekt erfasst werden können.

Alternativ oder zusätzlich kann eine Stimulationsbetriebsart der implantierbaren Anordnung auf eine für eine His-Bündel-Stimulation spezifische Betriebsart eingestellt werden. Dies kann beispielsweise durch eine Vorgabe bestimmter Parameterbereiche erfolgen, die unterschiedliche Funktionen der implantierbaren Anordnung (insbesondere Stimulation, Detektion und/oder Speicherung der detektierten oder durchgeführten Ereignisse) betreffen.

Alternativ oder zusätzlich kann eine Stimulationsfrequenz (Simulationsrate) einer Stimulation eingestellt werden, die von einer zur His-Bündel-Stimulation geeigneten Elektrode abgegeben werden kann.

Alternativ oder zusätzlich kann auch eine spezifische His-Bündel-Stimulations-Einstellung eines zusätzlichen Elektrodenanschlusses der Stimulationseinheit erfolgen, an den eine zur Stimulation des His-Bündels eines menschlichen oder tierischen Herzens vorgesehene Elektrode zusätzlich angeschlossen ist (sogenannter His-Bündel-Backup-Stimulationsanschluss).

In einer Variante veranlasst das Programm den Prozessor dazu, zusätzlich den nachfolgend erläuterten Schritt durchzuführen. Bei diesem zusätzlichen Schritt wird ein Betriebszustand der implantierbaren Anordnung in einen Schutzmodus umgeschaltet, wenn der Schutzmodus durch ein externes Signal aktiviert wurde. Im Gegensatz zu einem Sicherheitsmodus, der durch eine geräteinterne Diagnose der Funktionsfähigkeit der implantierbaren Anordnung aktiviert wird, handelt es Schutzmodus also um einen durch ein externes Signal aktivierbaren Modus. Beispielsweise könnte Schutzmodus durch eine Programmiergerät-Notfunktion aktiviert werden. Dies kann beispielsweise von einem Arzt erfolgen, wenn er während einer Untersuchung feststellt, dass die implantierbare Anordnung nicht in der bestimmungsgemäßen Art und Weise funktioniert.

Der Schutzmodus stellt gemäß einer Ausführungsform eine standardisierte, einheitliche sichere Betriebsart für den Stimulator dar, die für alle Patienten mit einem zur HIS-Stimulation ausgelegten Implantat angewendet werden kann. Im Unterschied dazu kann der Sicherheitsmodus zum Beispiel von einer individuellen Geräteeinstellung abhängen und patientenspezifisch konfiguriert sein.

Der Schutzmodus wird dann zumindest aus einem ersten und einem zweiten Schutzmodus ausgewählt.

Der erste Schutzmodus wird - analog zum ersten Sicherheitsmodus - dann ausgewählt, wenn die vorherige Überprüfung ergeben hat, dass keine zur Stimulation des His-Bündels eines menschlichen oder tierischen Herzens vorgesehene Elektrode an die Stimulationseinheit angeschlossen ist. Der erste Schutzmodus verursacht eine Überführung der implantierbaren Anordnung in einem ersten Schutzbetrieb, in dem die implantierbare Anordnung keine spezifischen Anforderungen einer His-Bündel-Stimulation erfüllt. Denn wenn keine His-Bündel-Elektrode an die Stimulationseinheit der Anordnung angeschlossen ist, muss keine Rücksicht auf spezifische Anforderungen einer His-Bündel-Stimulation genommen werden.

Demgegenüber wird der zweite Schutzmodus dann ausgewählt, wenn die vorherige Überprüfung, ob eine zur His-Bündel-Stimulation geeignete Elektrode an die Stimulationseinheit angeschlossen ist, ergeben hat, dass eine derartige Elektrode vorhanden ist. Der zweite Schutzmodus aktiviert einen zweiten Schutzbetrieb der implantierbaren Anordnung, in dem die implantierbare Anordnung in der Lage ist, spezifische Anforderungen einer His-Bündel-Stimulation zu erfüllen. Dabei ist der zweite Schutzmodus mit dem zweiten Sicherheitsmodus vergleichbar. Daher sind insbesondere sämtliche Varianten, die in der vorliegenden Anmeldung in Bezug auf den zweiten Sicherheitsmodus beschrieben werden, auch unmittelbar auf den zweiten Schutzmodus übertragbar.

Daher ist der zweite Schutzbetrieb in einer Variante durch mindestens eine der folgenden Konfigurationen gekennzeichnet: eine His-Bündel-Stimulations-Einstellung eines Elektrodenanschlusses der Stimulationseinheit, an den eine zur Stimulation eines His-Bündels eines menschlichen oder tierischen Herzens vorgesehene Elektrode angeschlossen ist; eine Stimulationsenergie einer von einer zur Stimulation eines His-Bündels eines menschlichen oder tierischen Herzens vorgesehenen Elektrode abzugebenden Stimulation; eine Detektionsempfindlichkeit der Detektionseinheit für eine Detektion His-Bündel-spezifischer Signale; eine Stimulationsbetriebsart der implantierbaren Anordnung; eine Stimulationsfrequenz einer von einer zur Stimulation eines His-Bündels eines menschlichen oder tierischen Herzens vorgesehenen Elektrode abzugebenden Stimulation; und eine His-Bündel-Stimulations-Einstellung eines zweiten Elektrodenanschlusses der Stimulationseinheit, an den eine zweite Elektrode zur Stimulation eines menschlichen oder tierischen Herzens angeschlossen ist. Bei der zweiten Elektrode kann es sich beispielsweise um eine rechts- oder linksventrikuläre Stimulationselektrode handeln, und bei dem zweiten Elektrodenanschluss entsprechend um den Anschluss für eine rechts- oder linksventrikuläre Stimulationselektrode.

In einer Variante ist es möglich, dass die Konfiguration des zweiten Sicherheitsbetriebs (und/oder des zweiten Schutzbetriebs) in Abhängigkeit einstellbarer Programmierparameter festgelegt oder individuell angepasst werden kann. Zu diesem Zweck ist es möglich, dass ein Benutzer die entsprechenden Programmierparameter nach den Bedürfnissen eines Patienten, dem die implantierbare Anordnung implantiert wurde oder wird, anpasst. Dann ist es möglich, auf patientenspezifische Besonderheiten einzugehen und somit einen individuellen Sicherheitsbetrieb bzw. Schutzbetrieb der Anordnung zu ermöglichen.

In einer Variante sind in der implantierbaren Anordnung Informationen gespeichert, auf deren Grundlage die Konfiguration des zweiten Sicherheitsbetriebs (und/oder des zweiten Schutzbetriebs) automatisch bestimmt wird. Das heißt, dass in dieser Variante keine Einstellmöglichkeiten von Parametern in Bezug auf die Konfiguration des zweiten Sicherheitsbetriebs vorgesehen sind. Bei dieser Variante wird das Risiko einer versehentlichen Fehleinstellung bestimmter Parameter ausgeräumt, da der zweite Sicherheitsbetrieb (und/oder der zweite Schutzbetrieb) stets mit voreingestellten Parametern durchgeführt wird.

In einer Variante weist die implantierbare Anordnung eine Datenfernübertragungseinheit auf. Das Programm kann den Prozessor in dieser Variante dazu veranlassen, eine Aktivierung des Sicherheitsmodus mittels der Datenfernübertragungseinheit an ein Fernüberwachungssystem zu übertragen. Dann kann im Fernüberwachungssystem erkannt werden, dass bei der implantierbaren Anordnung ein Störzustand detektiert wurde, der eine Aktivierung des Sicherheitsmodus zur Folge hatte. Der Patient, dem die implantierbare Anordnung implantiert wurde, und/oder dessen Arzt können dann informiert werden, dass eine Überprüfung der Funktion der implantierbaren Anordnung erforderlich ist.

In einer Variante wird auf einem Programmiergerät, das zur Programmierung der implantierbaren Anordnung eingesetzt werden kann, angezeigt, dass der Sicherheitsmodus aktiviert wurde. Dabei kann auch angezeigt werden, welcher Sicherheitsmodus aktiviert wurde. So kann besonders einfach überprüft werden, ob die Einstellungen, die für die entsprechende implantierbare Anordnung in Bezug auf das Vorhandensein einer zur His-Bündel-Stimulation geeigneten Elektrode hinterlegt sind, tatsächlich korrekt sind. Darüber hinaus lässt sich so der Erfolg einer Aktivierung oder Inaktivierung des Sicherheitsmodus besonders einfach überprüfen.

Ein Aspekt der vorliegenden Erfindung betrifft ein Verfahren zur Steuerung des Betriebs einer implantierbaren Anordnung zur Stimulation eines menschlichen oder tierischen Herzens gemäß den vorherigen Erläuterungen. Dieses Verfahren ist durch die nachfolgend erläuterten Schritte gekennzeichnet.

In einem Verfahrensschritt wird mittels einer Diagnoseeinheit zur Überprüfung von Funktionsparametern der implantierbaren Anordnung erkannt, ob mindestens ein Störzustand der implantierbaren Anordnung vorliegt.

In einem weiteren Verfahrensschritt wird überprüft, ob eine Elektrode, die zur Stimulation eines His-Bündels eines menschlichen oder tierischen Herzens vorgesehen ist, an eine Stimulationseinheit der implantierbaren Anordnung angeschlossen ist. Diese Stimulationseinheit ist dabei zum Stimulieren eines His-Bündels oder eines anderen kardialen Bereichs eines menschlichen oder tierischen Herzens ausgelegt.

In einem weiteren Verfahrensschritt wird ein Betriebszustand der implantierbaren Anordnung in einen Sicherheitsmodus umgeschaltet, wenn ein Störzustand erkannt wurde. Der Sicherheitsmodus kann dabei zumindest aus einem ersten und einem zweiten Sicherheitsmodus ausgewählt werden.

Der erste Sicherheitsmodus wird dann ausgewählt, wenn das zuvor erfolgte Überprüfen ergeben hat, dass keine Elektrode an die Stimulationseinheit angeschlossen ist, die zur Stimulation eines His-Bündels eines menschlichen oder tierischen Herzens vorgesehen ist. Der erste Sicherheitsmodus führt zur Aktivierung eines ersten Sicherheitsbetriebs der implantierbaren Anordnung, in dem die implantierbare Anordnung keine spezifischen Anforderungen einer His-Bündel-Stimulation erfüllt. Denn dies ist regelmäßig nicht erforderlich, wenn gar keine zur His-Bündel-Stimulation vorgesehene Elektrode an die Stimulationseinheit der Anordnung angeschlossen ist.

Demgegenüber wird der zweite Sicherheitsmodus ausgewählt, wenn die zuvor erfolgte Überprüfung ergeben hat, dass eine Elektrode, die zur Stimulation eines His-Bündels eines menschlichen oder tierischen Herzens geeignet ist, an die Stimulationseinheit angeschlossen ist. Der zweite Sicherheitsmodus bewirkt einen zweiten Sicherheitsbetrieb der implantierbaren Anordnung, in dem die implantierbare Anordnung in der Lage ist, spezifische Anforderungen einer His-Bündel-Stimulation zu erfüllen.

Ein Aspekt der vorliegenden Erfindung betrifft ein Computerprogrammprodukt mit einem computerlesbaren Code, der einen Prozessor dazu veranlasst, die nachfolgend erläuterten Schritte auszuführen, wenn er auf dem Prozessor ausgeführt wird.

In einem Verfahrensschritt wird mittels einer Diagnoseeinheit zur Überprüfung von Funktionsparametern der implantierbaren Anordnung erkannt, ob mindestens ein Störzustand der implantierbaren Anordnung vorliegt.

In einem weiteren Verfahrensschritt wird überprüft, ob eine Elektrode, die zur Stimulation eines His-Bündels eines menschlichen oder tierischen Herzens vorgesehen ist, an eine Stimulationseinheit der implantierbaren Anordnung angeschlossen ist. Diese Stimulationseinheit ist dabei zum Stimulieren eines His-Bündels oder eines anderen kardialen Bereichs eines menschlichen oder tierischen Herzens ausgelegt.

In einem weiteren Verfahrensschritt wird ein Betriebszustand der implantierbaren Anordnung in einen Sicherheitsmodus umgeschaltet, wenn ein Störzustand erkannt wurde. Der Sicherheitsmodus kann dabei zumindest aus einem ersten und einem zweiten Sicherheitsmodus ausgewählt werden.

Der erste Sicherheitsmodus wird dann ausgewählt, wenn das zuvor erfolgte Überprüfen ergeben hat, dass keine Elektrode an die Stimulationseinheit angeschlossen ist, die zur Stimulation eines His-Bündels eines menschlichen oder tierischen Herzens vorgesehen ist. Der erste Sicherheitsmodus führt zur Aktivierung eines ersten Sicherheitsbetriebs der implantierbaren Anordnung, in dem die implantierbare Anordnung keine spezifischen Anforderungen einer His-Bündel-Stimulation erfüllt. Denn dies ist regelmäßig nicht erforderlich, wenn gar keine zur His-Bündel-Stimulation vorgesehene Elektrode an die Stimulationseinheit der Anordnung angeschlossen ist.

Demgegenüber wird der zweite Sicherheitsmodus ausgewählt, wenn die zuvor erfolgte Überprüfung ergeben hat, dass eine Elektrode, die zur Stimulation eines His-Bündels eines menschlichen oder tierischen Herzens geeignet ist, an die Stimulationseinheit angeschlossen ist. Der zweite Sicherheitsmodus bewirkt einen zweiten Sicherheitsbetrieb der implantierbaren Anordnung, in dem die implantierbare Anordnung in der Lage ist, spezifische Anforderungen einer His-Bündel-Stimulation zu erfüllen.

Ein weiterer Aspekt der vorliegenden Erfindung betrifft ein Verfahren zur Behandlung eines menschlichen oder tierischen Patienten, der eine solche Behandlung benötigt. Diese Behandlung erfolgt mittels einer implantierbaren Anordnung zur Stimulation eines menschlichen oder tierischen Herzens, insbesondere mittels einer implantierbaren Anordnung gemäß den vorherigen Erläuterungen. Eine derartige implantierbare Anordnung weist eine Stimulationseinheit zum Stimulieren eines His-Bündels oder eines anderen kardialen Bereichs eines menschlichen oder tierischen Herzens auf. Sie weist ferner eine Detektionseinheit zum Detektieren eines elektrischen Signals desselben Herzens auf. Schließlich ist eine derartige Anordnung mit einer Diagnoseeinheit ausgestattet, die zur Überprüfung von Funktionsparametern der implantierbaren Anordnung dient. Das medizinische Verfahren weist die nachfolgend erläuterten Schritte auf:
In einem Verfahrensschritt wird mittels der Diagnoseeinheit erkannt, ob mindestens ein Störzustand der implantierbaren Anordnung vorliegt.

In einem weiteren Verfahrensschritt wird überprüft, ob eine Elektrode, die zur Stimulation eines His-Bündels eines menschlichen oder tierischen Herzens vorgesehen ist, an die Stimulationseinheit angeschlossen ist.

In einem weiteren Verfahrensschritt wird ein Betriebszustand der implantierbaren Anordnung in einen Sicherheitsmodus umgeschaltet, wenn ein Störzustand erkannt wurde. Der Sicherheitsmodus kann dabei zumindest aus einem ersten und einem zweiten Sicherheitsmodus ausgewählt werden.

Der erste Sicherheitsmodus wird dann ausgewählt, wenn das zuvor erfolgte Überprüfen ergeben hat, dass keine Elektrode an die Stimulationseinheit angeschlossen ist, die zur Stimulation eines His-Bündels eines menschlichen oder tierischen Herzens vorgesehen ist. Der erste Sicherheitsmodus führt zur Aktivierung eines ersten Sicherheitsbetriebs der implantierbaren Anordnung, in dem die implantierbare Anordnung keine spezifischen Anforderungen einer His-Bündel-Stimulation erfüllt. Denn dies ist regelmäßig nicht erforderlich, wenn gar keine zur His-Bündel-Stimulation vorgesehene Elektrode an die Stimulationseinheit der Anordnung angeschlossen ist.

Demgegenüber wird der zweite Sicherheitsmodus ausgewählt, wenn die zuvor erfolgte Überprüfung ergeben hat, dass eine Elektrode, die zur Stimulation eines His-Bündels eines menschlichen oder tierischen Herzens geeignet ist, an die Stimulationseinheit angeschlossen ist. Der zweite Sicherheitsmodus bewirkt einen zweiten Sicherheitsbetrieb der implantierbaren Anordnung, in dem die implantierbare Anordnung in der Lage ist, spezifische Anforderungen einer His-Bündel-Stimulation zu erfüllen.

Nachfolgend wird - bei entsprechendem medizinischem Bedarf - das menschliche oder tierische Herz des Patienten, dem die implantierbare Anordnung implantiert wurde, mittels der implantierbaren Anordnung stimuliert. Die Stimulation wird dabei in Ausübung des Sicherheitsbetriebs der implantierbaren Anordnung ausgeführt, der durch den zuvor ausgewählten Sicherheitsmodus vorgegeben ist.

Sämtliche Varianten und alternativen Ausgestaltungen, die in Zusammenhang mit der implantierbaren Anordnung beschrieben wurden, sind beliebig miteinander kombinierbar und auf die beschriebenen Verfahren und das beschriebene Computerprogrammprodukt übertragbar. Ferner sind die beschriebenen Varianten der Verfahren beliebig miteinander kombinierbar und auf die jeweils anderen Verfahren sowie auf das Computerprogrammprodukt und die implantierbare Anordnung übertragbar. In gleicher Weise sind die beschriebenen Varianten des Computerprogrammprodukts beliebig miteinander kombinierbar und auf die beschriebenen Verfahren und die beschriebene implantierbare Anordnung übertragbar.

Details von Aspekten der vorliegenden Erfindung sollen anhand von Ausführungsbeispielen und Figuren näher erläutert werden. Es zeigen:
- Fig. 1: eine schematische Darstellung unterschiedlicher Elektrodenkonfigurationen zum Anschluss einer zur His-Bündel-Stimulation geeigneten Elektrode an eine implantierbare Anordnung zur Stimulation des menschlichen oder tierischen Herzens und
- Fig. 2: ein schematisches Ablaufdiagramm eines Verfahrens zur Steuerung des Betriebs einer implantierbaren Anordnung zur Stimulation des menschlichen oder tierischen Herzens.

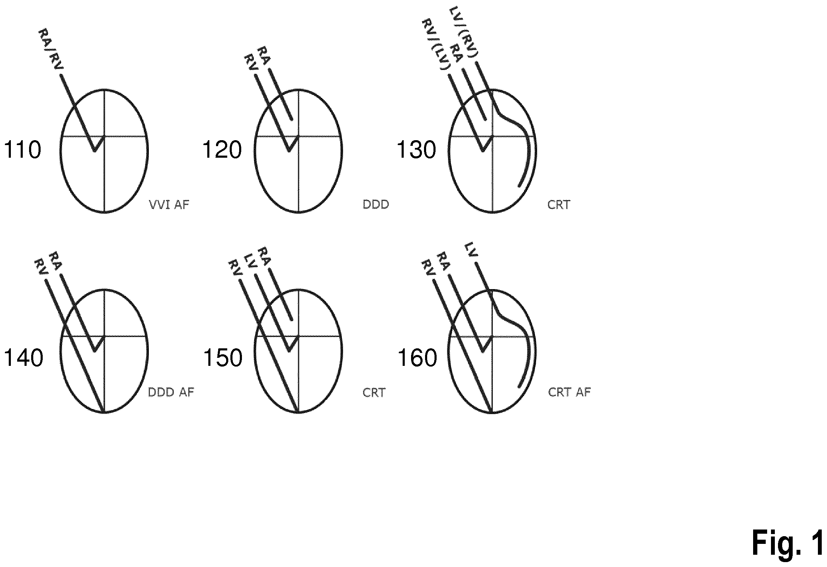

Die Fig. 1 zeigt in schematischer Darstellung verschiedene Elektrodenkonfigurationen, die zum Anschluss einer Elektrode, die zur Stimulation des His-Bündels eines menschlichen oder tierischen Herzens geeignet ist, an ein Gerät zur Stimulation dieses Herzens möglich sind.

In einer ersten Elektronenkonfiguration 110 erfolgt eine Einkammer-Stimulation. Zu diesem Zweck ist die zur His-Bündel-Stimulation geeignete Elektrode an einen Elektrodenanschluss für eine Elektrode zur Stimulation des rechten Atriums RA oder zur Stimulation des rechten Ventrikels RV angeschlossen. Ein derartiger Schrittmacher kann auch als Einkammerschrittmacher bzw. Bedarfsschrittmacher bezeichnet werden. Er wird zumindest im Sicherheitsmodus typischerweise in einem Modus betrieben, in dem eine Stimulation im rechten Ventrikel und eine Detektion im rechten Ventrikel folgt und eine inhibierende Betriebsart ausgeführt wird (VVI). Das heißt, der Schrittmacher gibt nur dann Stimulationspulse ab, wenn keine herzeigene Aktivität festgestellt werden kann. Mittels der His-Bündel-Elektrode ist in diesem Modus ist eine rechtsventrikuläre oder biventrikuläre Stimulation möglich.

Wenn die zur His-Bündel-Stimulation vorgesehene Elektrode an den RA-Anschluss eines solchen Schrittmachers angeschlossen ist und ein solcher Schrittmacher in einen VVI-Sicherheitsmodus fällt, ist keine ausreichende His-Bündel-Stimulation möglich. Daher eignet sich die vorliegend beschriebene Erfindung gerade zu Verbesserungen von Herzschrittmachern mit der ersten Elektronenkonfiguration 110. Aber auch bei weiteren Elektronenkonfigurationen, die nachfolgend vorgestellt werden, ergibt sich typischerweise eine Unterversorgung des Patienten durch die Auswahl herkömmlicher Sicherheitsmodi. Auch hier sorgt die vorliegend beanspruchte Erfindung für Abhilfe, indem sie durch einen speziell für die His-Bündel-Stimulation ausgestalteten Sicherheitsmodus sorgt und dessen Aktivierung beim Anschluss einer His-Bündel-Elektrode ermöglicht.

In einer zweiten Elektrodenkonfiguration 120 ist die zur Stimulation des His-Bündels eingesetzte Elektrode an den Anschluss des rechten Ventrikels RV angeschlossen. Zusätzlich ist noch eine arterielle Elektrode an den Anschluss für das rechte Atrium RA angeschlossen. Ein solcher Schrittmacher kann im DDD-Modus (duale Stimulation (also im Atrium und im Ventrikel), duale Detektion (also im Atrium und im Ventrikel) in dualer Betriebsart (also getriggert oder inhibiert) betrieben werden. Mittels der His-Bündel-Elektrode ist auch in diesem Modus ist eine rechtsventrikuläre oder biventrikuläre Stimulation möglich. Dabei ist eine sequenzielle Stimulation des Atriums und des His-Bündels möglich.

In einer dritten Elektrodenkonfiguration 130 ist die zur Stimulation des His-Bündels geeignete Elektrode an den Anschluss für den rechten Ventrikel RV oder den linken Ventrikel LV des Herzschrittmachers angeschlossen. Demgegenüber ist eine zur Stimulation des linken Ventrikels vorgesehene Elektrode an den noch freien Anschluss zu Stimulation des linken Ventrikels LV oder des rechten Ventrikels RV angeschlossen. Darüber hinaus ist eine atriale Elektrode an den Anschluss für das rechte Atrium RA angeschlossen. Bei einem solchen Stimulationsgerät handelt es sich insbesondere um ein Gerät zur kardialen Resynchronisation (CRT). Es ermöglicht eine biventrikuläre Stimulation sowie eine kardiale Resynchronisation mit optionaler His-Bündel-Stimulation.

In einer vierten Elektrodenkonfiguration 140 ist die zur Stimulation des His-Bündels vorgesehene Elektrode an den Anschluss für das rechte Atrium RA eines Schrittmachers angeschlossen. Darüber hinaus ist eine Elektrode zur Stimulation des rechten Ventrikels an den entsprechenden Anschluss für den rechten Ventrikel RV angeschlossen. Ein solcher Schrittmacher kann einen RV-Sicherheitsmodus ausführen und ist insbesondere zum Betrieb im DDD-Modus geeignet.

In einer fünften Elektrodenkonfiguration 150 ist die zur His-Bündel-Stimulation vorgesehene Elektrode an den Anschluss für den linken Ventrikel LV angeschlossen. Darüber hinaus ist eine atriale Elektrode an den Anschluss für das rechte Atrium RA angeschlossen und eine ventrikuläre Elektrode an den Anschluss für den rechten Ventrikel RV. Auch bei einem solchen Gerät handelt es sich insbesondere um ein CRT-Gerät, das eine rechtsventrikuläre oder biventrikuläre Stimulation ermöglicht und zudem eine optionale His-Bündel-Stimulation bei der Durchführung einer kardialen Resynchronisation ermöglicht.

In einer sechsten Elektrodenkonfiguration 160 ist die zur His-Bündel-Stimulation vorgesehene Elektrode wiederum an den Anschluss für das rechte Atrium RA angeschlossen. Darüber hinaus sind zwei ventrikuläre Elektroden vorgesehen, von denen die im rechten Ventrikel angeordnete Elektrode an den Anschluss für den rechten Ventrikel RV und die linksventrikuläre Elektrode an den Anschluss für den linken Ventrikel LV angeschlossen ist. Eine solche Elektrodenkonfiguration eignet sich abermals besonders für CRT-Geräte, die eine biventrikuläre Stimulation und eine kardiale Resynchronisation mit optionaler His-Bündel-Stimulation ausführen können.

Bei den verschiedenen Stimulationsvorrichtungen, deren Elektrodenkonfigurationen in der Fig. 1 dargestellt sind, ist eine Implementierung der vorliegend beanspruchten Erfindung sinnvoll und angedacht. Denn bei allen diesen Elektrodenkonfigurationen ergibt sich das Problem, dass eine Unterversorgung des Patienten eintreten kann, wenn herkömmliche Sicherheitsmodi ausgewählt werden. Durch die Möglichkeit der Auswahl eines auf die spezifischen Anforderungen einer His-Bündel-Stimulation zugeschnittenen Sicherheitsmodus ist es möglich, eine derartige Unterversorgung zu vermeiden, wenn eine zur His-Bündel-Stimulation geeignete Elektrode an dem entsprechenden Stimulationsgerät angeschlossen ist.

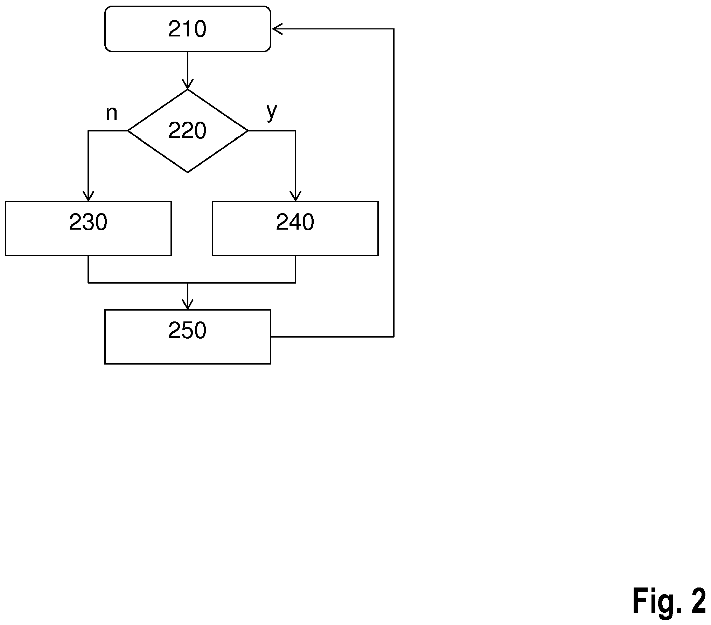

Die Fig. 2 zeigt ein schematisches Ablaufdiagramm eines Verfahrens zur Steuerung des Betriebs einer implantierbaren Anordnung zur Stimulation des menschlichen oder tierischen Herzens, die den erfindungsgemäßen zweiten Sicherheitsmodus ausführen kann (nachfolgend als Stimulationsvorrichtung bezeichnet).

Zunächst ist diese Stimulationsvorrichtung in einer regulären Betriebsart 210 aktiv. Wird durch einen zyklisch durchgeführten Selbsttest mit einer entsprechenden Diagnoseeinheit ein Fehlerzustand erkannt, erfolgt eine Überprüfung 220, ob eine zur Stimulation des His-Bündels geeignete Elektrode an die Stimulationsvorrichtung angeschlossen ist. Dies kann beispielsweise über die Abfrage einer internen Information sein, die beim Anschluss einer zur His-Bündel-Stimulation geeigneten Elektrode in der Stimulationsvorrichtung hinterlegt wird.

Ergibt die Überprüfung 220, dass keine zur His-Bündel-Stimulation geeignete Elektrode an die Stimulationsvorrichtung angeschlossen ist, wird ein erster Sicherheitsmodus 230 ausgewählt, durch den nachfolgend ein erster Sicherheitsbetrieb der Stimulationsvorrichtung durchgeführt wird.

Ergibt die Überprüfung 220 hingegen, dass eine zur His-Bündel-Stimulation vorgesehene Elektrode an die Stimulationsvorrichtung angeschlossen ist, wird ein zweiter Sicherheitsmodus 240 ausgewählt, der die Stimulationsvorrichtung in einen zweiten Sicherheitsbetrieb überführt. Dieser zweite Sicherheitsbetrieb ist speziell auf die Bedürfnisse einer His-Bündel-Stimulation zugeschnitten, sodass eine ausreichende His-Bündel-Stimulation auch in diesem Sicherheitsmodus möglich ist.

Nach einer Wiederherstellungsprozedur 250, in der insbesondere der Fehlerzustand beseitigt wird, der überhaupt zur Überführung der Stimulationsvorrichtung in einen der Sicherheitsmodi geführt hat, wird die Stimulationsvorrichtung wieder in ihre regulären Betriebsart 210 überführt.

## Patentansprüche

1. Implantierbare Anordnung zur Stimulation eines menschlichen oder tierischen Herzens, aufweisend einen Prozessor, eine Speichereinheit, eine Stimulationseinheit zum Stimulieren eines His-Bündels oder eines anderen kardialen Bereichs eines menschlichen oder tierischen Herzens, eine Detektionseinheit zum Detektieren eines elektrischen Signals desselben Herzens und eine Diagnoseeinheit zur Überprüfung von Funktionsparametern der implantierbaren Anordnung,
**dadurch gekennzeichnet,**
**dass** die Speichereinheit ein computerlesbares Programm aufweist, das den Prozessor dazu veranlasst, die folgenden Schritte auszuführen, wenn es auf dem Prozessor ausgeführt wird:
a) Erkennen mittels der Diagnoseeinheit, ob mindestens ein Störzustand der implantierbaren Anordnung vorliegt,
b) Überprüfen (220), ob eine zur Stimulation eines His-Bündels eines menschlichen oder tierischen Herzens vorgesehene Elektrode an die Stimulationseinheit angeschlossen ist,
c) Umschalten eines Betriebszustands der implantierbaren Anordnung in einen Sicherheitsmodus, wenn ein Störzustand erkannt wurde, wobei der Sicherheitsmodus zumindest aus einem ersten Sicherheitsmodus (230) und einem zweiten Sicherheitsmodus (240) ausgewählt wird, wobei
i) der erste Sicherheitsmodus (230) ausgewählt wird, wenn das in Schritt b) erfolgte Überprüfen (220) ergeben hat, dass keine zur Stimulation eines His-Bündels eines menschlichen oder tierischen Herzens vorgesehene Elektrode an die Stimulationseinheit angeschlossen ist, wobei der erste Sicherheitsmodus (230) einen ersten Sicherheitsbetrieb der implantierbaren Anordnung bedingt, in dem die implantierbare Anordnung keine spezifischen Anforderungen einer His-Bündel-Stimulation erfüllt,
ii) der zweite Sicherheitsmodus (240) ausgewählt wird, wenn das in Schritt b) erfolgte Überprüfen (220) ergeben hat, dass eine zur Stimulation eines His-Bündels eines menschlichen oder tierischen Herzens vorgesehene Elektrode an die Stimulationseinheit angeschlossen ist, wobei der zweite Sicherheitsmodus (240) einen zweiten Sicherheitsbetrieb der implantierbaren Anordnung bedingt, in dem die implantierbare Anordnung in der Lage ist, spezifische Anforderungen einer His-Bündel-Stimulation zu erfüllen.

2. Implantierbare Anordnung nach Anspruch 1, **dadurch gekennzeichnet, dass** für das Umschalten der Anordnung in den Sicherheitsmodus benötigte Informationen in einem nicht-flüchtigen Speicher, einem redundanten Speicher oder einem wiederherstellbaren Speicher der implantierbaren Anordnung hinterlegt sind.

3. Implantierbare Anordnung nach Anspruch 1 oder 2, **dadurch gekennzeichnet, dass** das Überprüfen (220), ob eine zur Stimulation eines His-Bündels eines menschlichen oder tierischen Herzens vorgesehene Elektrode an die Stimulationseinheit angeschlossen ist, anhand einer in der Speichereinheit gespeicherten Information erfolgt.

4. Implantierbare Anordnung nach einem der vorhergehenden Ansprüche, **dadurch gekennzeichnet, dass** die Diagnoseeinheit dafür vorgesehen und eingerichtet ist, den Störzustand anhand mindestens eines der folgenden Ereignisse zu erkennen: ein Speicherfehler, ein Softwarelaufzeitfehler, ein Softwarestatusfehler, ein erkannter Cyberangriff, ein Parameterfehler, ein Aktivieren eines Reset-Zustands der implantierbaren Anordnung, eine Batterieerschöpfung und eine externe elektromagnetische Störung der implantierbaren Anordnung.

5. Implantierbare Anordnung nach einem der vorhergehenden Ansprüche, **dadurch gekennzeichnet, dass** der zweite Sicherheitsbetrieb durch mindestens eine der folgenden Konfigurationen gekennzeichnet ist: eine His-Bündel-Stimulations-Einstellung eines Elektrodenanschlusses der Stimulationseinheit, an den eine zur Stimulation eines His-Bündels eines menschlichen oder tierischen Herzens vorgesehene Elektrode angeschlossen ist; eine Stimulationsenergie einer von einer zur Stimulation eines His-Bündels eines menschlichen oder tierischen Herzens vorgesehenen Elektrode abzugebenden Stimulation; eine Detektionsempfindlichkeit der Detektionseinheit für eine Detektion His-Bündel-spezifischer Signale; eine Stimulationsbetriebsart der implantierbaren Anordnung; eine Stimulationsfrequenz einer von einer zur Stimulation eines His-Bündels eines menschlichen oder tierischen Herzens vorgesehenen Elektrode abzugebenden Stimulation; und eine His-Bündel-Stimulations-Einstellung eines zweiten Elektrodenanschlusses der Stimulationseinheit, an den eine zweite Elektrode zur Stimulation eines menschlichen oder tierischen Herzens angeschlossen ist.

6. Implantierbare Anordnung nach einem der vorhergehenden Ansprüche, **dadurch gekennzeichnet, dass** das Programm den Prozessor dazu veranlasst, zusätzlich folgenden Schritt durchzuführen:
d) Umschalten eines Betriebszustands der implantierbaren Anordnung in einen Schutzmodus, wenn der Schutzmodus durch ein externes Signal aktiviert wurde, wobei der Schutzmodus zumindest aus einem ersten und einem zweiten Schutzmodus ausgewählt wird, wobei
i) der erste Schutzmodus ausgewählt wird, wenn das in Schritt b) erfolgte Überprüfen (220) ergeben hat, dass keine zur Stimulation eines His-Bündels eines menschlichen oder tierischen Herzens vorgesehene Elektrode an die Stimulationseinheit angeschlossen ist, wobei der erste Schutzmodus einen ersten Schutzbetrieb der implantierbaren Anordnung bedingt, in dem die implantierbare Anordnung keine spezifischen Anforderungen einer His-Bündel-Stimulation erfüllt,
ii) der zweite Schutzmodus ausgewählt wird, wenn das in Schritt b) erfolgte Überprüfen ergeben hat, dass eine zur Stimulation eines His-Bündels eines menschlichen oder tierischen Herzens vorgesehene Elektrode an die Stimulationseinheit angeschlossen ist, wobei der zweite Schutzmodus einen zweiten Schutzbetrieb der implantierbaren Anordnung bedingt, in dem die implantierbare Anordnung in der Lage ist, spezifische Anforderungen einer His-Bündel-Stimulation zu erfüllen.

7. Implantierbare Anordnung nach einem der vorhergehenden Ansprüche, **dadurch gekennzeichnet, dass** der zweite Schutzbetrieb durch mindestens eine der folgenden Konfigurationen gekennzeichnet ist: eine His-Bündel-Stimulations-Einstellung eines Elektrodenanschlusses der Stimulationseinheit, an den eine zur Stimulation eines His-Bündels eines menschlichen oder tierischen Herzens vorgesehene Elektrode angeschlossen ist; eine Stimulationsenergie einer von einer zur Stimulation eines His-Bündels eines menschlichen oder tierischen Herzens vorgesehenen Elektrode abzugebenden Stimulation; eine Detektionsempfindlichkeit der Detektionseinheit für eine Detektion His-Bündel-spezifischer Signale; eine Stimulationsbetriebsart der implantierbaren Anordnung; eine Stimulationsfrequenz einer von einer zur Stimulation eines His-Bündels eines menschlichen oder tierischen Herzens vorgesehenen Elektrode abzugebenden Stimulation; und eine His-Bündel-Stimulations-Einstellung eines zusätzlichen Elektrodenanschlusses der Stimulationseinheit, an den eine zur Stimulation eines His-Bündels eines menschlichen oder tierischen Herzens vorgesehene Elektrode zusätzlich angeschlossen ist.

8. Implantierbare Anordnung nach einem der vorhergehenden Ansprüche, **dadurch gekennzeichnet, dass** das Programm den Prozessor dazu veranlasst, die Konfiguration des zweiten Sicherheitsbetriebs in Abhängigkeit einstellbarer Programmierparameter festzulegen oder individuell anzupassen.

9. Implantierbare Anordnung nach einem der Ansprüche 1 bis 7, **dadurch gekennzeichnet, dass** das Programm den Prozessor dazu veranlasst, die Konfiguration des zweiten Sicherheitsbetriebs in Abhängigkeit von in der implantierbaren Anordnung gespeicherten Informationen automatisch zu bestimmen.

10. Implantierbare Anordnung nach einem der vorhergehenden Ansprüche, **dadurch gekennzeichnet, dass** die implantierbare Anordnung eine Datenfernübertragungseinheit aufweist, wobei das Programm den Prozessor dazu veranlasst, eine Aktivierung des Sicherheitsmodus mittels der Datenfernübertragungseinheit an ein Fernüberwachungssystem zu übertragen.

11. Verfahren zur Steuerung des Betriebs einer implantierbaren Anordnung zur Stimulation eines menschlichen oder tierischen Herzens gemäß einem der vorhergehenden Ansprüche, **gekennzeichnet durch** die folgenden Schritte:
a) Erkennen mittels einer Diagnoseeinheit zur Überprüfung von Funktionsparametern der implantierbaren Anordnung, ob mindestens ein Störzustand der implantierbaren Anordnung vorliegt,
b) Überprüfen (220), ob eine zur Stimulation eines His-Bündels eines menschlichen oder tierischen Herzens vorgesehene Elektrode an eine Stimulationseinheit zum Stimulieren eines His-Bündels oder eines anderen kardialen Bereichs eines menschlichen oder tierischen Herzens angeschlossen ist,
c) Umschalten eines Betriebszustands der implantierbaren Anordnung in einen Sicherheitsmodus, wenn ein Störzustand erkannt wurde, wobei der Sicherheitsmodus zumindest aus einem ersten Sicherheitsmodus (230) und einem zweiten Sicherheitsmodus (240) ausgewählt wird, wobei
i) der erste Sicherheitsmodus (230) ausgewählt wird, wenn das in Schritt b) erfolgte Überprüfen (220) ergeben hat, dass keine zur Stimulation eines His-Bündels eines menschlichen oder tierischen Herzens vorgesehene Elektrode an die Stimulationseinheit angeschlossen ist, wobei der erste Sicherheitsmodus (230) einen ersten Sicherheitsbetrieb der implantierbaren Anordnung bedingt, in dem die implantierbare Anordnung keine spezifischen Anforderungen einer His-Bündel-Stimulation erfüllt,
ii) der zweite Sicherheitsmodus (240) ausgewählt wird, wenn das in Schritt b) erfolgte Überprüfen (220) ergeben hat, dass eine zur Stimulation eines His-Bündels eines menschlichen oder tierischen Herzens vorgesehene Elektrode an die Stimulationseinheit angeschlossen ist, wobei der zweite Sicherheitsmodus (240) einen zweiten Sicherheitsbetrieb der implantierbaren Anordnung bedingt, in dem die implantierbare Anordnung in der Lage ist, spezifische Anforderungen einer His-Bündel-Stimulation zu erfüllen.

12. Computerprogrammprodukt mit einem computerlesbaren Code, der einen Prozessor dazu veranlasst, die folgenden Schritte auszuführen, wenn er auf dem Prozessor ausgeführt wird:
a) Erkennen mittels einer Diagnoseeinheit zur Überprüfung von Funktionsparametern einer implantierbaren Anordnung zur Stimulation eines menschlichen oder tierischen Herzens, ob mindestens ein Störzustand der implantierbaren Anordnung vorliegt,
b) Überprüfen (220), ob eine zur Stimulation eines His-Bündels eines menschlichen oder tierischen Herzens vorgesehene Elektrode an eine Stimulationseinheit zum Stimulieren eines His-Bündels oder eines anderen kardialen Bereichs eines menschlichen oder tierischen Herzens angeschlossen ist,
c) Umschalten eines Betriebszustands der implantierbaren Anordnung in einen Sicherheitsmodus, wenn ein Störzustand erkannt wurde, wobei der Sicherheitsmodus zumindest aus einem ersten Sicherheitsmodus (230) und einem zweiten Sicherheitsmodus (240) ausgewählt wird, wobei
i) der erste Sicherheitsmodus (230) ausgewählt wird, wenn das in Schritt b) erfolgte Überprüfen (220) ergeben hat, dass keine zur Stimulation eines His-Bündels eines menschlichen oder tierischen Herzens vorgesehene Elektrode an die Stimulationseinheit angeschlossen ist, wobei der erste Sicherheitsmodus (230) einen ersten Sicherheitsbetrieb der implantierbaren Anordnung bedingt, in dem die implantierbare Anordnung keine spezifischen Anforderungen einer His-Bündel-Stimulation erfüllt,
ii) der zweite Sicherheitsmodus (240) ausgewählt wird, wenn das in Schritt b) erfolgte Überprüfen (220) ergeben hat, dass eine zur Stimulation eines His-Bündels eines menschlichen oder tierischen Herzens vorgesehene Elektrode an die Stimulationseinheit angeschlossen ist, wobei der zweite Sicherheitsmodus (240) einen zweiten Sicherheitsbetrieb der implantierbaren Anordnung bedingt, in dem die implantierbare Anordnung in der Lage ist, spezifische Anforderungen einer His-Bündel-Stimulation zu erfüllen.

13. Verfahren zur Behandlung eines menschlichen oder tierischen Patienten, der eine solche Behandlung benötigt, mittels einer implantierbaren Anordnung zur Stimulation eines menschlichen oder tierischen Herzens, wobei die implantierbare Anordnung eine Stimulationseinheit zum Stimulieren eines His-Bündels oder eines anderen kardialen Bereichs eines menschlichen oder tierischen Herzens, eine Detektionseinheit zum Detektieren eines elektrischen Signals desselben Herzens und eine Diagnoseeinheit zur Überprüfung von Funktionsparametern der implantierbaren Anordnung aufweist, wobei das Verfahren die folgenden Schritte umfasst:
a) Erkennen mittels der Diagnoseeinheit, ob mindestens ein Störzustand der implantierbaren Anordnung vorliegt,
b) Überprüfen (220), ob eine zur Stimulation eines His-Bündels eines menschlichen oder tierischen Herzens vorgesehene Elektrode an die Stimulationseinheit angeschlossen ist,
c) Umschalten eines Betriebszustands der implantierbaren Anordnung in einen Sicherheitsmodus, wenn ein Störzustand erkannt wurde, wobei der Sicherheitsmodus zumindest aus einem ersten Sicherheitsmodus (230) und einem zweiten Sicherheitsmodus (240) ausgewählt wird, wobei
i) der erste Sicherheitsmodus (230) ausgewählt wird, wenn das in Schritt b) erfolgte Überprüfen (220) ergeben hat, dass keine zur Stimulation eines His-Bündels eines menschlichen oder tierischen Herzens vorgesehene Elektrode an die Stimulationseinheit angeschlossen ist, wobei der erste Sicherheitsmodus (230) einen ersten Sicherheitsbetrieb der implantierbaren Anordnung bedingt, in dem die implantierbare Anordnung keine spezifischen Anforderungen einer His-Bündel-Stimulation erfüllt,
ii) der zweite Sicherheitsmodus (240) ausgewählt wird, wenn das in Schritt b) erfolgte Überprüfen (220) ergeben hat, dass eine zur Stimulation eines His-Bündels eines menschlichen oder tierischen Herzens vorgesehene Elektrode an die Stimulationseinheit angeschlossen ist, wobei der zweite Sicherheitsmodus (240) einen zweiten Sicherheitsbetrieb der implantierbaren Anordnung bedingt, in dem die implantierbare Anordnung in der Lage ist, spezifische Anforderungen einer His-Bündel-Stimulation zu erfüllen,
d) Ausführen einer Stimulation des menschlichen oder tierischen Herzens in dem durch den ausgewählten Sicherheitsmodus vorgegebenen Sicherheitsbetrieb der implantierbaren Anordnung.
